Europäisches Patentamt

European Patent Office

Office européen des brevets

(19)

(11) Veröffentlichungsnummer : **0 189 430**
**B1**

(12) **EUROPÄISCHE PATENTSCHRIFT**

(45) Veröffentlichungstag der Patentschrift :
03.01.90

(21) Anmeldenummer : 85902478.8

(22) Anmeldetag : 30.05.85

(86) Internationale Anmeldenummer :
PCT/DE 85/00189

(87) Internationale Veröffentlichungsnummer :
WO/8505634 (19.12.85 Gazette 85/27)

(51) Int. Cl.⁵ : **C 12 P 17/18** // (C12P17/18,
C12R1:645)

(54) **VERFAHREN ZUR HERSTELLUNG VON FESTUCLAVIN.**

(30) Priorität : 01.06.84 DE 3420955

(43) Veröffentlichungstag der Anmeldung :
06.08.86 Patentblatt 86/32

(45) Bekanntmachung des Hinweises auf die Patenterteilung : 03.01.90 Patentblatt 90/01

(84) Benannte Vertragsstaaten :
AT BE CH DE FR GB IT LI LU NL SE

(56) Entgegenhaltungen :
Chemical Abstracts, vol. 97, No. 11, 13 September
1982, (Columbus, Ohio, US) Janardhanan K.K. et al. :
"Studies on Clavicepsparasitic on Panicum species
in India", page 408, abstract No. 88317v

(73) Patentinhaber : SCHERING AKTIENGESELLSCHAFT
Berlin und Bergkamen
Müllerstrasse 170/178 Postfach 65 03 11
D-1000 Berlin 65 (DE)

(72) Erfinder : WILKE, Detlef
Landwehr 6
D-3015 Wennigsen/Deister (DE)
Erfinder : WEBER, Alfred
Schützallee 56
D-1000 Berlin 37 (DE)

EP 0 189 430 B1

## Beschreibung

Die Erfindung betrifft das in dem Patentanspruch gekennzeichnete Verfahren.

Festuclavin (6,8-Dimethyl-ergolin) ist bekanntlich eine Vorstufe zur Biosynthese pharmakologisch wirksamer Ergotalkaloide und kann unter anderem zur Herstellung pharmakologisch wirksamer $N_1$-Alkyl-4,8-dimethyl-ergolinen verwendet werden. (CSSR-Patent 189 485, referiert in C.A. 96, 1982, 143 148x). Nach den bisher bekannten Verfahren wird es aber auf fermentativem Wege nur im Gemisch mit anderen Ergotalkaloiden erhalten. Demzufolge ist seine Herstellung und Isolierung sehr aufwendig.

Es wurde nun gefunden, daß ein Pilzstamm Claviceps paspali Festuclavin in hohen Ausbeuten und praktisch frei von anderen Ergotalkaloiden in das Kulturmedium ausscheidet. Dieser Stamm Claviceps paspali wurde aus einem Mutterkorn einer in Argentinien wild wachsenden Grassorte der Gattung Paspalum isoliert. Er hat die interne Bezeichnung SCHERING, MBCE 12 426 und ist bei der deutschen Sammlung für Mikroorganismen unter der Nummer DSM 2 838 hinterlegt. Der Stamm bildet auf Agarmedien mit Glucose oder Saccharose als Kohlenstoffquelle und Asparagin, Ammoniumsuccinat oder komplexen Stickstoffquellen wie Pepton flache, schnellwüchsige Kolonien die aus Hyphen, Konidien und kurzen, verdickten und stark vakuolisierten Zellen bestehen.

Die Hyphen haben einen Durchmesser von 4 bis 5 μm. Die Konidien sind oval und haben eine Breite von 6 bis 10 μm und eine Länge von 10 bis 20 μm. Die Kolonien sind grauweiß, kompakt, und zeigen eine flechtenartig gekräuselte Oberfläche und einen unregelmäßig ausgefransten Rand. Der Koloniendurchmesser beträgt nach 10 Tagen Kulturzeit 1 bis 2 cm und nach 35 Tagen Kulturzeit 4 bis 5 cm.

Das erfindungsgemäße Verfahren wird unter Bedingungen durchgeführt, die man üblicherweise zur Anzucht von Pilzkulturen für eine Stoffwechselsynthese anwendet. So werden zunächst in allgemein üblichen Vorversuchen die günstigsten Fermentationsbedingungen, wie z. B. die Auswahl des günstigsten Nährmediums, der technischen Bedingungen wie Temperatur, Belüftung pH-Wert und der optimalen Zeiten für die Germination und die Entwicklung des Mikroorganismus ermittelt.

Als Kohlenstoffquelle kann für das Fermentationsmedium beispielsweise Glucose oder Saccharose verwendet werden. Als Stickstoffquelle dient unter anderem Asparagin, Ammoniumsuccinat oder komplexe Substanzen wie Pepton. Ferner enthält das Medium die nötigen Wuchsstoffe (beispielsweise Hefeextrakt) und Mineralstoffe (Kalium-, Magnesium-, Kalzium-, Eisen- und Zink-Kationen, sowie Sulfat, Phosphat, Nitrat und Chlorid-Anionen) in der üblicherweise angewendeten Konzentration.

Die Fermentation kann ein- oder zweistufig erfolgen, wobei das für die Vorkultur verwendete Medium mit dem der Hauptkultur identisch sein oder von diesem verschieden sein kann.

Zu Beginn der Fermentation wird der pH-Wert des Mediums vorzugsweise in einem Bereich von 4 bis 6 eingestellt. Die Züchtungstemperatur liegt im Bereich von etwa 10 bis 35 °C, vorzugsweise im Bereich von 20 bis 50 °C. Die Kulturbedingungen sind streng aerob. Die optimale Fermentationszeit wird durch Analyse des gebildeten Festuclavins in üblicher Weise ermittelt.

Nach erfolgter Fermentation wird das gebildete Festuclavin in an sich bekannter Weise isoliert, beispielsweise · indem man die Fermentationsansätze mit einem nicht mit Wasser mischbaren organischen Lösungsmittel, wie Ethylacetat, Methylisobutylketon, Dichlormethan, Chloroform oder Tetrachlorethan extrahiert, die Extrakte einengt und das erhaltene Rohprodukt durch Chromatographie und/oder Kristallisation reinigt.

Die nachfolgenden Ausführungsbeispiele dienen zur Erläuterung des erfindungsgemäßen Verfahrens.

Beispiel 1

Claviceps paspali DSM 2838 wird auf einem Nährmedium angezüchtet, welches folgende Komponenten enthält:

Saccharose (100 g/l), Asparagin (10 g/l), Hefeextrakt (0,1 g/l), Kaliumdihydrogenphosphat (250 mg/l), Magnesiumsulfat-Heptahydrat (250 mg/l), Kaliumchlorid (120 mg/l), Calciumnitrat-Tetrahydrat (1 g/l), Eisensulfat-Heptahydrat (20 mg/l), Zinksulfat-Heptahydrat (15 mg/l), Agar (18 g/l). Das Nährmedium ist auf pH 5,1 eingestellt. Die Anzuchtskultur wird 5 bis 20 Tage lang bei 30 °C im Brutschrank aufbewahrt.

Ein ca. 1 cm² großes Mycelstück wird mittels eines Ultraturrax unter sterilen Bedingungen in 5 ml physiologischer Kochsalzlösung zerkleinert und damit 50 ml eine Vorkultur enthaltend Saccharose (100 g/l), Pepton (20 g/l), Kaliumdihydrogenphosphat (1 g/l), Magnesiumsulfat-Heptahydrat (250 mg/l), die sich in einem 500 ml Erlenmeyerkolben befindet, beimpft und auf einem Rundschüttler 4 Tage lang bei 24 °C und 220 Umdrehungen pro Minute kultiviert.

5 ml der so erhaltenen Vorkultur werden in 50 ml eines Mediums enthaltend Saccharose (100 g/l), Asparagin (10 g/l), Hefeextrakt (0,1 g/l), Kaliumdihydrogenphosphat (250 mg/l), Magesiumsulfat-Heptahydrat (250 mg/l), Kaliumchlorid (120 mg/l), Calciumnitrat-Tetrahydrat (1 g/l), Eisensulfat-Heptahydrat (20 mg/l) und Zinksulfat (15 mg/l) — eingestellt auf pH 5,1 — die sich in einem 500 ml Erlenmeyerkolben befindet, überführt und 7 Tage lang bei 24 °C auf einem Rundschüttler mit 240 Umdrehungen pro Minute geschüttelt.

Dann wird das Kulturmedium abfiltriert und der Gehalt an festuclavin mittels der van Urk's-Reaktion (Mikrochim. Acta, 1959, 619-630) fotometrisch ermittelt. Die Konzentration des Kulturfiltrates beträgt 2,28 g/l.

Beispiel 2

Unter den Bedingungen des Beispiels 1 aber nach Austausch des Vor- und Hauptkulturmediums gegeneinander erhält man nach 9 Tagen Kulturzeit im Medium der Hauptkultur 2,07 g pro Liter Festuclavin.

## Patentanspruch

Verfahren zur Herstellung von Festuclavin, dadurch gekennzeichnet, daß man den Mikroorganismus Claviceps paspali DSM 2 838 kultiviert und das gebildete Festuclavin nach Beendigung der Fermentation isoliert.

## Claim

Process for the preparation of festuclavin, characterised in that the microorganism Claviceps paspali DSM 2 838 is cultivated and the festuclavin formed is isolated when fermentation is complete.

## Revendication

Procédé de préparation de la festuclavine caractérisé en ce qu'on cultive le micro-organisme Claviceps paspali DSM 2 838 et, lorsque la fermentation est terminée, on isole la festuclavine formée.